# EUROPEAN PATENT APPLICATION

(11) **EP 1 489 419 A1**
(43) Date of publication of application: **22.12.2004**
(21) Application number: 03745017.8
(22) Date of filing: 27.03.2003
(51) Int. Cl.: G01N 33/542, G01N 33/543

(54) **FLUORESCENT POLARIZATION METHOD, KIT USED THEREFOR AND BIOSENSOR**

(30) Priority: 27.03.2002 JP 2002090036
(71) Applicant: MATSUSHITA ELECTRIC INDUSTRIAL CO., LTD., Kadoma-shi, Osaka 571-8501 (JP)
(72) Inventor: KITAWAKI, Fumihisa, Katano-shi, Osaka 576-0021 (JP); KAWAMURA, Tatsurou, Kyotanabe-shi, Kyoto 610-0351 (JP)
(74) Representative: Grünecker, Kinkeldey, Stockmair & Schwanhäusser Anwaltssozietät
(86) International application number: PCT/JP2003/003911
(87) International publication number: WO 2003/081243

(57) **Abstract**

A fluorescence polarization assay is a method for measuring a subject substance, which includes the steps of: (a) preparing a first solid support body on which a binding substance capable of specifically binding to the subject substance is immobilized and a fluorescent labeled substance which is capable of specifically binding to the subject substance and is labeled with a fluorescent dye; (b) bringing the fluorescent labeled substance and the binding substance into contact with the subject substance; and (c) measuring the polarization degree of fluorescent light from the fluorescent labeled substance.

## Description

### TECHNICAL FIELD

The present invention relates to a method for analyzing a subject substance contained in a sample.

### BACKGROUND ART

Immunoassy is a method for performing measurement using an antigen-antibody reaction. Up until now, immunoassays based on various measurement principles have been reported. For example, there are assays, such as enzyme immunoassay, fluorometry, and luminometry, which require separation of an antigen-antibody complex from an unreacted substance (referred to as "BF separation": BF is an abbreviation of binding/free), and other assays, such as turbidimetry, nephelometry, latex agglutination assay, which require no BF separation. Each of those assays is a method for optically detecting an antigen-antibody reaction.

As for immunoassays, an assay of the BF separation requiring type such as enzyme immunoassay, fluorometry and luminometry needs a washing process for removing free antigen and antibody which are not involved in an antigen-antibody reaction. Moreover, if a washing process is incorporated in a system of an apparatus and also automated, the operativity of an assay can be improved. However, such a washing-process-incorporated apparatus is expensive.

An assay of the non-BF-separation requiring type such as turbidimetry, nephelometry and latex agglutination assay, among immunoassays, has relatively good operativity, compared to the BF separation requiring type assay. However, since a complex including an antigen and an antibody generated through an antigen-antibody reaction is detected with scattering light or transmitted light in the non-BF-separation requiring type assay, the assay is not suitable for measuring items which require high sensitivity.

In recent years, an immunoassay for detecting an antigen-antibody reaction using polarized fluorescent light (which will be referred to as a "fluorescence polarization assay") has attracted attention. The fluorescence polarization assay does not require BF separation, and therefore no washing operation is needed. Moreover, since it is basically fluorescent light that is measured, high sensitivity can be obtained. Thus, the fluorescence polarization assay has more advantages than the other assays described above.

Studies on use of the phenomenon called "fluorescence polarization" (also called "fluorescence depolarization") with biopolymers started in 1950 and full-scale applied studies of fluorescence polarization covering a wide area in the field of biochemistry, biology or the like have been carried out since the late 1970s.

An apparatus specially designed for measuring polarized fluorescent light (which will be herein referred to as a "fluorescence polarization measurement apparatus") will be briefly described. The fluorescence polarization measurement apparatus has a configuration obtained by further adding two polarizer plates to the same configuration of that of a known fluorometer. In the fluorescence polarization measurement apparatus, a tetra-transparent-quartz cell is used as a cell of a detection unit as in a regular fluorescence measurement. To measure fluorescent light in a visible range, a glass cell can be used.

With the fluorescence polarization measurement apparatus, a monochrome wave (monochrome light) is obtained from light emitted from a light source by a filter or a grating prism. Then, the obtained monochrome wave is polarized by a polarizer, so that excitation light is obtained. When a cell is exposed to the excitation light having passed through the polarizer, only molecules having a fluorescent dye in a solution absorb the excitation light. In the fluorescence polarization measurement apparatus, vertical and horizontal components of fluorescent light emitted during a relaxation time of characteristic fluorescent light of the fluorescent dye are detected, fluorescent light polarization is calculated based on Perrin's formula. In principle, a value for fluorescent light polarization is dependent on a difference of the rotational speed of molecules varying depending on the size of the molecules. For this reason, even if there is an influence of an impurity which interferes detection of fluorescent light, or the like, a value for fluorescent light intensity is not directly dependent on an antigen concentration. Therefore, except for the cases where fluorescent light is not detected at all, a measurement for a sample can be performed without requiring pre-treatment. Furthermore, BF separation is not required either. As can be understood from the description above, the fluorescence polarization assay is considered to be an immunoassay which can be carried out in a simple manner.

As an example of known immunoassay techniques using fluorescence polarization, there is an assay disclosed in Japanese Unexamined Patent Publication No. 11-81332. In these assays, an antibody which is capable of binding to a subject substance and is labeled with a fluorescent dye is used. In general, various types of immunoassays are used in clinical examinations, as have been described.

### Problems to be solved

However, since the known fluorescence polarization assay is a method for calculating the amount of a variation between molecular weights before and after an antigen-antibody reaction, it is difficult to measure the variation amount with high sensitivity if there is only a small variation between molecular weights before and after an antigen-antibody reaction, or if a relaxation time of fluorescent light emitted from a fluorescent dye is too short or too long.

The present invention has been devised in view of the above-described problems, and provides a fluorescence polarization assay, and a kit and a biosensor used for the same, which allow high sensitive measurement.

### DISCLOSURE OF INVENTION

A fluorescence polarization assay according to the present invention is a fluorescence polarization assay for measuring a subject substance which includes the steps of: (a) preparing a first solid support body on which a binding substance capable of specifically binding to a subject substance is immobilized, and a fluorescent labeled substance which is capable of specifically binding to the subject substance and is labeled with a fluorescent dye; (b) bringing the fluorescent labeled substance and the binding substance into contact with the subject substance; and (c) measuring the polarization degree of fluorescent light from the fluorescent labeled substance.

With the known assay which has been described, a binding substance capable of specifically binding to a subject substance is not immobilized on a solid support body. Because of this, a complex made of the subject substance and a fluorescent labeled substance is rotated. Thus, even if fluorescent light is irradiated, the fluorescent light is depolarized. Therefore, there are cases where the polarization degree of fluorescent light is not so largely varied. That is to say, there are cases where in measurement, the S/N ratio is increased and the sensitivity is reduced. With the fluorescence polarization assay of the present invention, on the other hand, when a subject substance is present in a sample, a fluorescent labeled substance and the subject substance specifically bind to each other in the step (b), and furthermore, the subject substance and a binding substance immobilized on a solid support body specifically bind to each other, so that a complex made of the subject substance, the fluorescent labeled substance and the binding substance is formed. Thus, a rotary motion of the fluorescent labeled substance is suppressed or stopped. Accordingly, the fluorescence polarization degree is largely varied and the variation amount can be expressed as a large measurement value.

The subject substance may be contained in a solution.

The fluorescence polarization assay may be configured so that in the step (c), the polarization degrees of fluorescent light emitted from the fluorescent labeled substance before and after the step (b) are measured.

The fluorescence polarization assay may be configured so that the fluorescence polarization assay further includes, before the step (b), a step (f) of bringing a solution which does not contain the subject substance into contact with the fluorescent labeled substance and the binding substance, in the step (c), the polarization degrees of fluorescent light from the fluorescent labeled substance after the steps (b) and (f) are measured.

It is preferable that the fluorescence polarization assay further includes a step (g) of obtaining the relationship between each of the polarization degrees of fluorescent light from the fluorescent labeled substance after the steps (b) and (f) and the concentration of the subject substance.

The fluorescence polarization assay may be configured so that the fluorescence polarization assay further includes a step (d) of preparing a second solid support body on which the binding substance is not immobilized, and a fluorescent labeled substance which is capable of specifically binding to the subject substance and is labeled with a fluorescent dye; and a step (e) of bringing the second solid support body and the fluorescent labeled substance into contact with the subject substance, the second solid support body is made of the same material as a material of the first solid support body from which the binding substance is removed, and in the step (c), the polarization degrees of fluorescent light from the fluorescent labeled substance after the steps of (b) and (e) are measured.

The first solid support body may be made of a microtiter plate.

The first solid support body may be made of a microparticle.

It is preferable that the fluorescent labeled substance is labeled with a fluorescent dye which emits fluorescent light whose relaxation time is 1 ns or more.

The fluorescent dye may be bound to a primary amino group, a secondary amino group, a tertiary amino group, a carboxyl group, a thiol group, a phenol group or a hydroxyl group contained in the binding substance.

The binding substance may be any one of an antibody, a receptor, a nucleic acid and an inhibitor.

The binding substance may be any one of a polyclonal antibody, a monoclonal antibody, a chimera antibody, an Fab antibody, an F(ab2) antibody, or an Fv antibody.

The subject substance may be an in vivo substance, a microorganism, or a virus.

The fluorescence polarization assay may be configured so that the binding substance is a first antibody, the fluorescent labeled substance is a second antibody labeled with a fluorescent dye, and the first and second antibodies recognize different epitopes, respectively.

A kit used in a fluorescence polarization assay for measuring a subject substance according to the present invention includes: a solid support body on which a binding substance capable of specifically binding to the subject substance is immobilized; and a fluorescent labeled substance which is capable of specifically binding to the subject substance and is labeled with a fluorescent dye.

With the kit of the present invention, when a subject substance is present in a sample, the fluorescent labeled substance and the subject substance specifically bind to each other, and furthermore, the subject substance and the binding substance immobilized on the solid support body specifically bind to each other, so that a complex made of the subject substance, the fluorescent labeled substance and the binding substance is formed. Thus, a rotary motion of the fluorescent labeled substance is suppressed or stopped. Accordingly, the fluorescence polarization degree is largely varied and the variation amount can be expressed as a large measurement value.

A biosensor according to the present invention includes a flow pass through which a solution containing a subject substance flows, and the flow pass includes: a sample introduction unit for introducing a solution containing a subject substance; a fluorescent labeled substance holding unit which is connected to the sample introduction unit and in which a fluorescent labeled substance which is capable of specifically binding to the subject substance and is labeled with a fluorescent dye is filled in a state where the fluorescent labeled substance can elute; and a binding substance holding unit which is connected to the fluorescent labeled substance holding unit and in which a binding substance capable of specifically binding to the subject substance is immobilized on a solid support body.

With the biosensor of the present invention, when a subject substance is present in a sample, the fluorescent labeled substance and the subject substance specifically bind to each other in the fluorescent labeled substance holding unit, and furthermore, the subject substance and the binding substance immobilized on the solid support body specifically bind to each other in the binding substance holding unit, so that a complex made of the subject substance, the fluorescent labeled substance and the binding substance is formed. Thus, a rotary motion of the fluorescent labeled substance is suppressed or stopped. Accordingly, the fluorescence polarization degree is largely varied and the variation amount can be expressed as a large measurement value.

The biosensor may be configured so that the solution is driven by capillary force, centrifugal force, or a potential difference through the flow pass.

The flow pass may be made of the solid support body, and the solid support body may be a capillary.

The biosensor may be configured so that the capillary is filled with a carrier.

The biosensor may be configured so that in the capillary, a solution flows freely.

The solid support body may be a material which impregnates a solvent of the solution containing the subject substance.

The biosensor may be configured so as to include at least two flow passes.

### BRIEF DESCRIPTION OF DRAWINGS

FIGS. **1(a)** through **1(c)** are schematic illustrations showing the principle on which the present invention is based.
FIG. **2** is a flowchart of a fluorescence polarization assay in accordance with the present invention.
FIG. **3** is a graph describing a summary of a biosensor which is one of embodiments of the present invention.
FIG. **4** illustrates a biosensor in accordance with the present invention and an exemplary configuration of a fluorescence polarization measuring apparatus using the biosensor.
FIGS. **5(a)** through **5(b)** are schematic diagrams showing a detection unit of the fluorescence polarization measuring apparatus in the exemplary configuration of FIG. **4**.
FIG. **6** is a graph showing measurement results of human chorionic gonadotropin in accordance with the present invention.

### BEST MODE FOR CARRYING OUT THE INVENTION

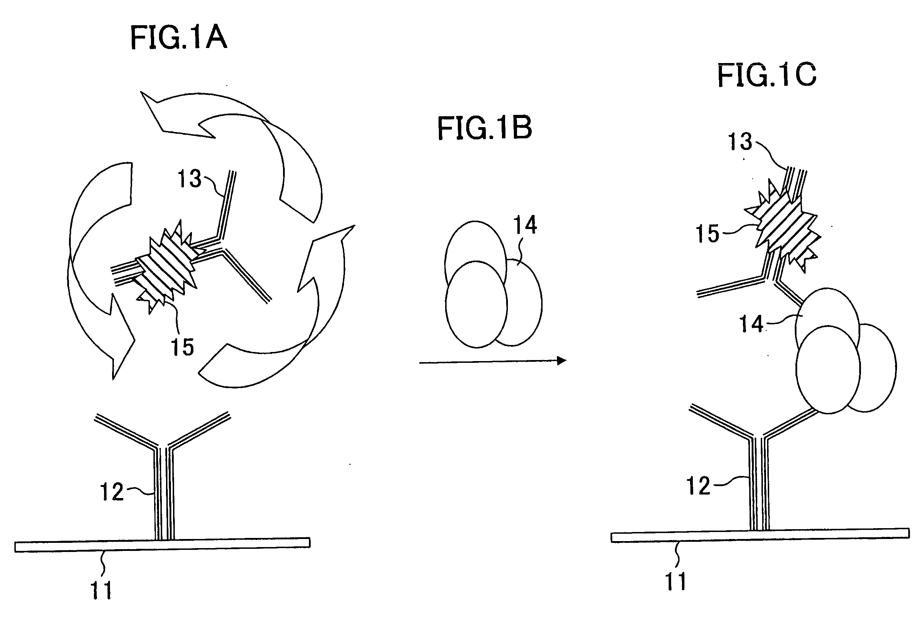

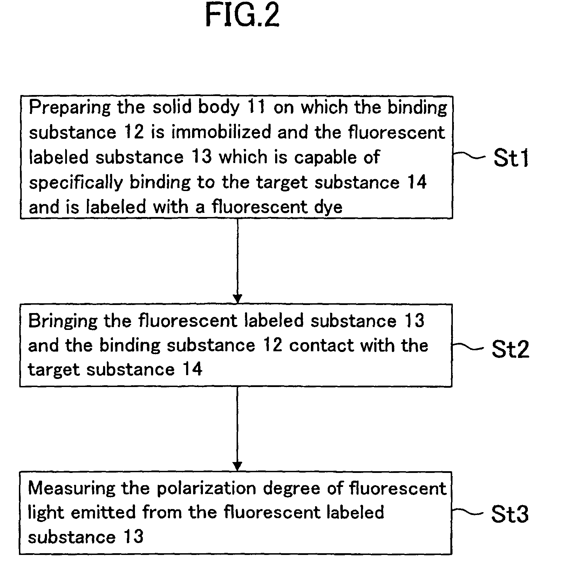

The present invention relates to a fluorescence polarization assay which is used in measurement of a subject substance contained in a sample and is performed on a solid phase on which a substance capable of specifically binding to the subject substance is immobilized. Hereinafter, embodiments of the present invention will be described with reference to FIGS. **1** and **2**. FIG. **1** shows schematic illustrations showing the principle on which the fluorescence polarization assay of the present invention is based. FIG. **2** is a flowchart of the fluorescence polarization assay of the present invention.

In general, a fluorescence polarization assay uses the phenomenon of depolariza.tion of fluorescent light due to a rotary motion of molecules in a solution. More specifically, as the size of molecules is smaller, the rotational speed of the molecules becomes greater, and thus the fluorescent light is largely depolarized. Accordingly, a value for the fluorescence polarization degree becomes small. In contrast, as the size of molecules is larger, the rotational speed of the molecules becomes slower, and thus not much of the fluorescence light is depolarized. Accordingly, the fluorescence polarization degree becomes large.

As shown FIG. **1,** in this embodiment, a solid support body **11** on which a binding substance **12** capable of specifically binding to a subject substance **14** is immobilized, and a fluorescent labeled substance **13** which is capable of specifically binding to the subject substance **14** and is labeled with a fluorescent dye are used. The subject substance **14** to which the fluorescent labeled substance **13** is bound is captured by the binding substance **12** immobilized on the solid support body **11**, so that a rotary motion of a complex of the fluorescent labeled substance **13** and the subject substance **14** is suppressed or stopped. This means that the complex of the fluorescent labeled substance **13** and the subject substance **14** in a solution is brought into a "stationary state" from a "motion state" by capturing the complex on a solid phase through a specific binding reaction to largely vary the fluorescence polarization degree. The variation amount of the fluorescence polarization degree indicates the concentration of the subject substance 14 which is bound to the immobilized binding substance **12** through a specific binding reaction.

The fluorescence polarization assay of this embodiment is a method for measuring the subject substance **14** and includes, as shown in FIG. **2,** Step St1 of preparing the solid support body **11** (typically an antibody) on which the binding substance **12** capable of specifically binding to the subject substance **14**, and the fluorescent labeled substance **13** which is capable of specifically binding to the subject substance **14** and is labeled with a fluorescent dye, Step St2 of bringing the fluorescent labeled substance **13** and the binding substance **12** into contact with the subject substance **14,** and Step St3 of measuring the polarization degree of fluorescent light from the fluorescent labeled substance **13.**

Each of the process steps will be described in detail.

In Step St1, as shown in FIG. **1(a),** when the subject substance **14,** the fluorescent labeled substance 13 is not captured by the binding substance **12** (typically an antibody) and is present in a solution. At this time, the fluorescent labeled substance 13 is rotated in the solution. Thus, even if polarized fluorescent light is irradiated, the fluorescent light is depolarized. Accordingly, a value for the fluorescence polarization degree is small.

In Step St2, as shown in FIG. **1(b),** a sample containing the subject substance **14** is added. Thus, a sandwich type complex with the subject substance **14** sandwiched between the fluorescent labeled substance 13 and the binding substance **12** is formed. Note that this process step may be performed before Step St1.

In Step St3, as shown in FIG. **1**(**c**), when the subject substance **14** is present, the fluorescent labeled substance **13** and the subject substance **14** which have been prepared in Step St1 specifically bind to each other, and furthermore, the subject substance **14** and the binding substance **12** immobilized on the solid support body **11** bind to each other, so that the sandwich type complex made of the subject substance **14,** the fluorescent labeled substance **13** and the binding substance **12** is formed. Thus, the rotary motion of the fluorescent labeled substance **13** is suppressed or stopped. Accordingly, the fluorescence polarization degree is largely varied and the variation amount can be expressed as a large measurement value. In this case, the concentration of the subject substance **14** in the sample is related to a difference between values for the fluorescence polarization degree when the subject substance **14** is not present and when the subject substance **14** is present.

As has been described, in a known assay (e.g., an assay disclosed in Japanese Unexamined Patent Publication No. 11-813332), a binding substance capable of specifically binding to a subject substance is not immobilized on a solid support body. Because of this, a rotary motion of a complex including the subject substance and a fluorescent labeled substance is caused. Thus, even if polarized fluorescent light is irradiated, the light is depolarized. Therefore, there might be cases where the fluorescence polarization degree is not so largely varied. That is to say, there might be cases where in measurement, the S/N ratio is increased and sensitivity is reduced.

However, in the fluorescence polarization assay of the present invention, as shown in FIG. **1**(**c**), when the subject substance **14** is present in a sample, the fluorescent labeled substance **13** prepared in Step St1 and the subject substance **14** specifically bind to each other, and furthermore, the subject substance **14** and the binding substance **12** immobilized on the solid support body **11** specifically bind to each other, so that a sandwich type complex made of the subject substance 14, the fluorescent labeled substance **13** and the binding substance **12** is formed. Thus, the rotary motion of the fluorescent labeled substance **13** is suppressed or stopped. Accordingly, the fluorescence polarization degree is largely varied and a variation amount for the fluorescence polarization degree can be expressed as a large measurement value.

In this embodiment, the fluorescence polarization assay is configured so that the polarization degree of fluorescent light from the fluorescent labeled substance **13** is measured before and after Step St2. However, the present invention is not limited thereto. For example, the fluorescence polarization assay may further include before Step St2 the pre-treatment step of bringing a solution which does not contain the subject substance **14** into contact with the fluorescent labeled substance **13** and the binding substance **12** and in Step St3, the polarization degree of fluorescent light from the fluorescent labeled substance **13** is measured after Step St2 and the pre-treatment step.

Furthermore, for example, the solid support body **11** on which the binding substance **12** is immobilized, and the fluorescent labeled substance **13** which is capable of specifically binding to the subject substance **14** and is labeled with a fluorescent dye are prepared and then measurement of the polarization degree of fluorescent light from the fluorescent labeled substance is started. Next, with the measurement continuously performed, a sample solution which contains the subject substance **14** is brought into contact with the fluorescent labeled substance **13** and the binding substance **12** after an arbitrary period of time from the time when the measurement of the fluorescence polarization degree started. Thus, it is possible to monitor how the fluorescence polarization degree has been varied with time since the time when the measurement of the fluorescence polarization degree started.

Moreover, the fluorescence polarization assay may be configured so that after solid support body (not shown) on which the binding substance **12** is not immobilized, and the fluorescent labeled substance **13** which is capable of specifically binding to the subject substance 14 and is labeled with a fluorescent dye have been prepared and then the solid support body on which the binding substance **12** is not immobilized and the fluorescent labeled substance **13** have been brought into contact with a sample solution which contains the subject substance **14**, the polarization degree of fluorescent light from the fluorescent labeled substance **13** is measured and also the polarization degree of fluorescent light from the fluorescent labeled substance **13** after Step St2 is measured.

In the fluorescence polarization assay of this embodiment, a kit including the solid support body **11** on which the binding substance **12** capable of specifically binding to the subject substance **14** is immobilized and the fluorescent labeled substance **13** capable of specifically binding to the subject substance 14 is used.

The solid support body **11** is, specifically, a measurement cell. As the measurement cell, a measurement cell which has been made to contain the fluorescent labeled substance **13** in advance may be used. With this kit, a measurement can be performed in a simple manner, i.e., by only placing the measurement cell in the fluorescence polarization measurement apparatus and introducing a sample into the measurement cell.

As a solid support body used for the fluorescence polarization assay and kit of this embodiment, a material capable of impregnating a solvent of a sample solution which contains a subject substance to be measured is preferably used. For example, a nitrocellulose membrane, a nitrocellulose acetate membrane, a glassfiber filter paper or the like is preferably used.

Moreover, as the solid support body used in the present invention, a microtiter plate is also preferably used. As the microtiter plate, a microtiter plate made of a polystyrene, polyvinyl, polycarbonate, polypropylene, silicon, glass or dextran material may be used.

As the solid support body used in the present invention, microparticles can be preferably used. As microparticles, for example, inorganic colloid, latex particles, magnetic particles or the like may be used.

To the solid support body used in the present invention, a binding substance which can specifically bind to a subject substance is immobilized through physisorption or chemisorption using a technique known by persons skilled in the art. A ligand such as a spacer well-known by persons skilled in the art may be introduced into the solid support body to immobilize the substance. As the spacer, a compound having a carbon chain with an arbitrary length may be used. Moreover, a ligand such as biotin which forms a complex with avidin is well-known by persons skilled in the art.

In this embodiment, as a substance (binding substance) to be immobilized on a solid support body, an arbitrary antibody, a receptor, a nucleic acid, an inhibitor or the like is used, as long as the substance specifically binds to a subject substance. Also, as a substance to be labeled with a fluorescent dye, an arbitrary antibody, a receptor, a nucleic acid, an inhibitor or the like is used as long as the substance specifically binds to the subject substance.

As an antibody used in this embodiment, a polyclonal antibody, a monoclonal antibody, a chimera antibody, a Fab anytibody, F(ab) 2 antibody or an Fv antibody may be used.

In this embodiment, a subject substance which can be measured, may be in vivo substances, microorganisms, viruses or the like. For example, in vivo substances includes a peptide, a protein, a lipid, a saccharide, a nucleic acid or the like. Microorganisms includes E. coli, Salmonella, Vibrio parahaemolyticus, Chlamydia, Helicobacter pylori or the like. Viruses includes Haemophilus influenzae, hepatitis C virus, HIV or the like.

As a fluorescent labeled substance used in this embodiment, any substances may be used as long as the substance specifically binds to a subject substance and is labeled with a fluorescent dye. In this embodiment, a binding substance labeled with a fluorescent dye is used. However, the present invention is not limited thereto. First and second antibodies which recognize different epitopes may be prepared and either one of the antibodies may be labeled with a fluorescent dye so that the labeled one is used as a fluorescent labeled substance and the other one is used as a binding substance as required.

Note that in the fluorescence polarization assay of this embodiment, the concentration of a fluorescent labeled substance is important. If a fluorescent labeled substance is present at a high concentration in a measurement system, the amount of the fluorescent labeled substance which is not captured (i.e., free substance) is larger than that of the fluorescent labeled substance captured by a binding substance immobilized on a solid support body with a subject substance sandwiched between the fluorescent labeled substance and the binding substance. If a fluorescence polarization measurement is performed in this state, a large variation in fluorescence polarization can not be obtained because the amount of the binding-free fluorescent labeled substance is large. Therefore, it is very much preferable to optimize the concentration of the fluorescent labeled substance in measuring a subject substance.

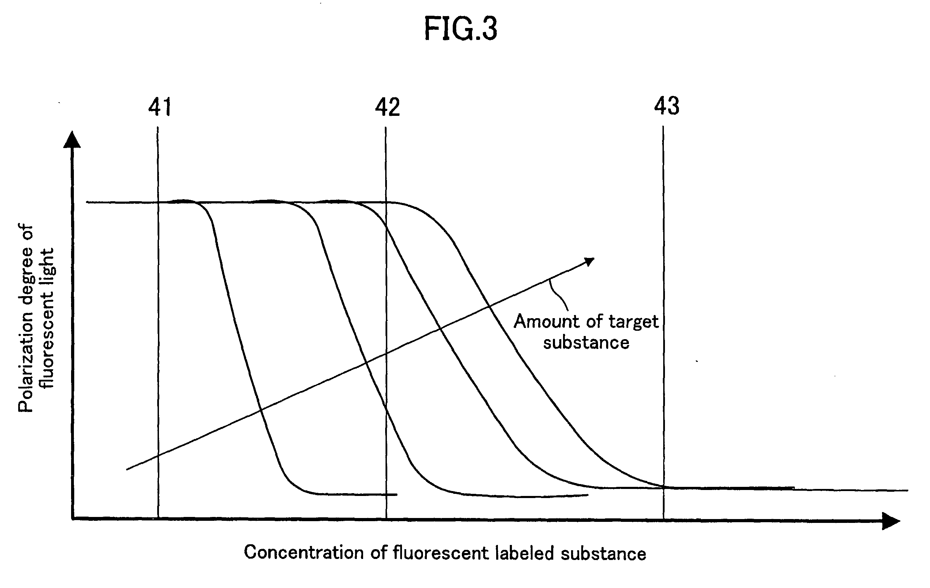

As an optimization technique, for example, an arbitrary amount of a subject substance is immobilized on a microtiter plate, each of serially diluted fluorescent labeled substance solutions is added thereto, and then the fluorescence polarization degree is measured. In general, as the concentration of fluorescent labeled substance is increased, the fluorescence polarization degree is reduced. Moreover, when the amount of the immobilized subject substance is increased, the concentration of the fluorescent labeled substance at which reduction in the fluorescence polarization degree is caused is increased. Assume that the results shown in FIG. 3 are obtained in a range of a subject substance concentration at which a measurement has to be guaranteed. If the fluorescent labeled substance concentration is set equal to or lower than the concentration level indicated by the line **4**1 in FIG. 3 and equal to or higher than the concentration level indicated by the line **42** in FIG. 3, variations in the fluorescence polarization degree are not observed. Therefore, it is preferable to set the fluorescent labeled substance concentration at around the concentration level indicated by the line **42** so that the fluorescence polarization degree varies according to the amount of a subject substance.

A fluorescent dye used for labeling a fluorescent labeled substance used in this embodiment is an important material for performing a measurement based on the fluorescence polarization assay as a principle. In general, characteristics of a fluorescent dye are exhibited by parameters such as an excitation wavelength, a fluorescent light wavelength, a stokes shift and a fluorescent light relaxation time. It is preferable that a fluorescent dye with characteristics defined by a fluorescent light relaxation time is used as a typical fluorescent dye of this embodiment and normally the range of the fluorescent light relaxation time is from about 0.1 ns to about 1000 ns. It is more preferable that a fluorescent dye having a fluorescence lifetime of about 1 ns or more is used. A fluorescent dye used in the present invention is selected taking the molecular weight of the fluorescent labeled substance which varies depending on its binding with a subject substance into consideration. This is because the polarization degree of fluorescent light emitted from the fluorescent labeled substance bound to the subject substance is proportional to the size of molecules, i.e., the rotational speed of molecules. In this embodiment, a fluorescent dye with which a fluorescent labeled substance having a small molecular weight, i.e., a great rotational speed in a solution can be obtained is preferably used, thus resulting in a larger variation in the fluorescence polarization degree between before and after the fluorescent labeled substance is captured. Examples of such a fluorescent dye are a fluorescein derivative, a dansyl derivative, a pyrene derivative, and a metal complex.

Moreover, a flurochrome used in this embodiment typically reacts with a primary amino group, a secondary amino group, a tertiary amino group, a carboxyl group, a thiol group, a phenol group or a hydroxyl group contained in a substance capable of specifically binding to the subject substance, so that the substance is labeled. Because of this, for example, a fluorescent dye obtained by introducing a functional group such as an isothiocyano group, a succimidyl group, a sulfonyl chloride group, an azide group, a thiol group and a maleimide group into one of the fluorescent dyes listed above according to a known introduction method may be used.

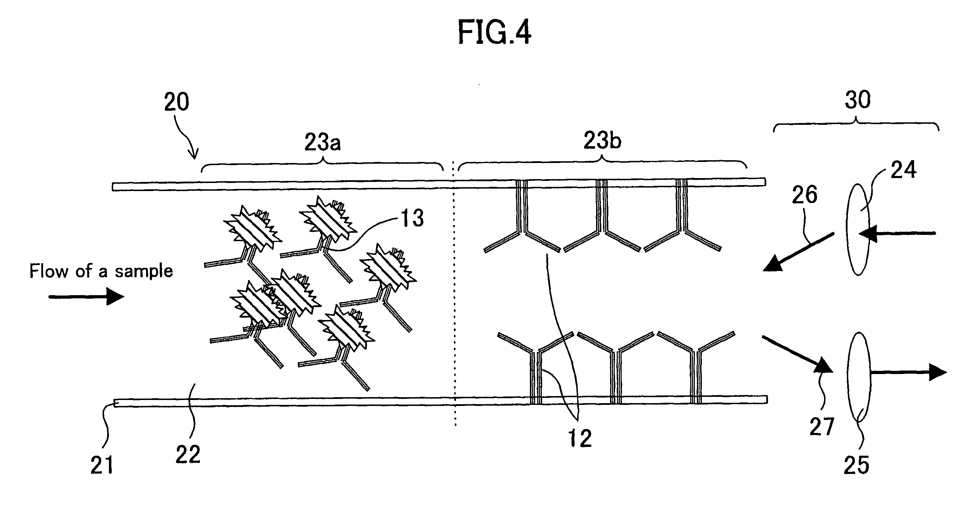

Next, a biosensor which uses the fluorescence polarization assay of this embodiment will be herein described with reference to FIG. **4**. FIG. **4** illustrates a biosensor acoording to this embodiment and an exemplary configuration of a fluorescence polarization measurement apparatus using the biosensor.

As shown in FIG. **4**, a biosensor **20** in accordance with this embodiment includes a solid support body **21** (a capillary in this embodiment) which is immobilized. The solid support body **21** includes a sample introduction unit **22**, a fluorescent labeled substance holding unit **23a** which is filled with a fluorescent labeled substance **13**, and a binding substance holding unit **23b** on which a binding substance **12** is immobilized. Each of the sample introduction unit **22**, the fluorescent labeled substance holding unit **23a** and the binding substance holding unit **23b** is a flow pass through which a sample solution containing a subject substance flows.

A fluorescence polarization measurement apparatus **30** for calculating a variation in the fluorescence polarization degree in the biosensor **20** includes an accommodation unit (not shown) for accommodating the biosensor **20,** further includes a polarizer plate **24** on a side of the biosensor which emits excitation light **26** and a polirizer plate **25** on a side thereof which is exposed with fluorescent light **27**, and has the function of rotating the polarizer plate **25** on the fluorescent light **27** side in measuring a variation in the fluorescence polarization degree.

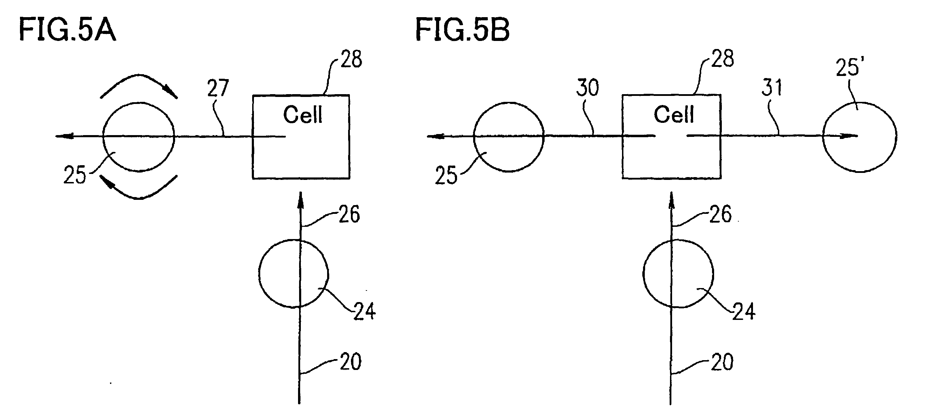

FIGS. **5(a)** and **5(b)** are schematic illustrations of a detection unit of the fluorescence polarization measurement apparatus according to the exemplary example shown in FIG. **4**. In each of FIGS. **5(a)** and **5(b)**, the binding substance holding unit **23b** of the biosensor **20** is schematically illustrated as a cell **28**. As shown in FIG. **5(a)**, the second polarizer plate **25** on the fluorescent light **27** side is rotated in the direction shown by the arrows when a variation in the fluorescence polarization degree is measured. Parallel and vertical components of fluorescent light emitted from the cell while the second polarizer plate **25** is rotated are sequentially monitored. Based on the monitored parallel and vertical components, the fluorescence polarization degree is calculated. Instead of this, as shown in FIG. **5(b)**, a detector which does not have the function of rotating a polarizer plate may be used as an apparatus for calculating a variation in the fluorescence polarization degree. In this case, as for fluorescent light emitted from the cell **28**, a parallel component **30** and a vertical component **31** thereof are monitored at the same time with two polarizer plates **25, 25'** disposed on the side of the apparatus which is exposed with fluorescent light. Based on the monitored parallel and vertical components **30** and **31**, the fluorescence polarization degree is calculated. Note that in FIG. **5**, the reference numerals **20, 24**, and **26** denote an incoming light, the excitation light side polarizer plate and an excitation light, respectively, as in FIG. **4**.

In the fluorescence polarization measurement apparatus **30** of this embodiment, a reaction unit for controlling temperature conditions to induce an antigen-antibody reaction, means for supplying the fluorescent labeled substance **13** to the fluorescent labeled substance holding unit **23a,** and means for calculating a variation in the fluorescence polarization degree may be integrated. Needless to say, the fluorescence polarization measurement apparatus **30** may be configured so that the fluorescence polarization measurement apparatus **30** includes a plurality of reaction units and means for measuring the fluorescence polarization measures the polarization degrees of fluorescent lights emitted from the plurality of reaction units at one time. Furthermore, the fluorescence polarization measurement apparatus **30** may be configured so as to further include means for calculating differences between the degrees of fluorescent lights measured at one time.

Moreover, it is also possible to make the fluorescence polarization measurement apparatus **30** into a biochip by further integrating the biosensor **20** in the fluorescence polarization measurement apparatus **30** in which the reaction units, the means for supplying the fluorescent labeled substance **13** and the means for calculating a variation in the fluorescence polarization degree.

A subject substance contained in a sample introduced from the sample introduction unit binds to the fluorescent labeled substance **13** through the fluorescent labeled substance holding unit **23**a, and further binds to the binding substance **12** (an antibody in this embodiment) immobilized on the solid support body **21** (a capillary in FIG. **4**). The fluorescence polarization measurement apparatus **30** detects a variation in the polarization degree of fluorescent light emitted from the binding substance holding unit 23b.

As a more simple assay than a known one, there is an assay using a dry chemistry technique. A dry chemistry technique is a technique for measuring a subject substance contained in a sample, in which a sample in a liquid form is dropped into a reagent stored in a dry state in a development layer matrix such as a film and a test paper so as to measure a subject substance contained in the sample. As a device, there are a single-layer type in which a reagent is supported by a single layer development layer matrix and a multi-layer type in which as a development layer matrix, a development layer, a reaction layer, a medicine layer or other layers are stacked. Characteristic of the assay using a dry chemistry technique are that, since a reagent is already supported on a development layer matrix, adjustment of the reagent is not necessary, only a small space is required for storing, and only a small amount of a subject substance is needed.

Immunochromatography assays (e.g., disclosed in Japanese Patent Publication No. 2890384) are typical assays using a dry chemistry technique. An immunochromatography assay is an assay which utilizes an antigen-antibody reaction and the capillary phenomenon. As a device, each of a first antibody which is immobilized and a second antibody labeled as a detected reagent is dry and supported on a carrier which is represented by a membrane filter. When an examination is performed, a specimen sample containing a subject substance (antigen) is added onto the device and is developed through the capillary phenomenon, so that a reaction unit is made to emit light through a sandwich type antigen-antibody reaction. Thus, an antigen is identified, whether or not the antigen is present is determined, or the amount of the antigen is measured.

In an immunochromatography assay, the process steps of reaction, washing and detection can be carried out by performing only one operation. Therefore, the operation thereof can be advantageously simple, and furthermore, a fast determination can be made. In recent years, as represented by pregnancy diagnostic test kits, the immunochromatography assay can be applied, because of its simpleness of operation, to point of care testing (POCT) which has been attracted attention in the field of clinical examination.

Immunochromatography assays known as simple assays used in clinical examination have an advantage of performing the process steps of reaction, washing and detection with only one operation. However, an antigen-antibody reaction requires a certain period of time, and thus a time which it takes for a liquid sample to be developed on a solid phase has to be increased. For this reason, an immunochromatography assay can be used only when a porous material such as a membrane is used. Furthermore, a porous material such as a membrane used in an immunochromatography assay is expensive and costs for a device become expensive.

Meanwhile, in the biosensor **20** of this embodiment, a material used as the solid support body **21** is not limited to a porous material such a membrane. Thus, the biosensor of this embodiment can be fabricated at a low cost. Therefore, the biosensor **20** can be preferably used in clinical examination, and more specifically in a POCT.

In the biosensor of this embodiment, a capillary is used as the solid support body **21**. In this embodiment, the biosensor includes the fluorescent labeled substance holding unit **23a** and the binding substance holding unit **23b** in the capillary, and the binding substance holding unit **23b** is made of, for example, a transparent material such as quartz glass and plastic so that a fluorescence polarization measurement can be performed. Moreover, as driving force for developing a sample in a liquid form in the capillary, for example, capillary force, centrifugal force and physical force such as a potential difference can be used. Moreover, it is also possible to fill the capillary with a carrier as necessary. As applicable carriers, a glass wool, a polyacrylamide gel, a microparticle or the like can be used. Needless to say, the capillary may be made without any carriers so that a solution can flow freely.

Moreover, a material used as the solid support body **21** of the biosensor **20** of this embodiment may be a material which has a rectangular shape (i.e., test strip) and is based on a porous material such as a nitrocellulose membrane, a nitrocellulose acetate membrane and a glassfiber filter paper. Capillary force is used as driving force for developing a liquid sample in such a porous material. The development direction may be either of the longitudinal direction and the lateral direction of the rectangular shape.

Note that in this embodiment, the biosensor including a flow pass has been described. However, needless to say, the biosensor may have the configuration which includes a plurality of flow passes so that multiple measurements are carried out at one time.

### Example

Hereinafter, the present invention will be described in detail using an example. The following example will be shown for the purpose of describing the present invention and in no way limits the present invention.

In the following procedure, a measurement is performed with human chorionic gonadotropin (hcg having a molecular weight of 37000) which is a urine substance, as a subject substance.

### 1. Preparation of a pyrene-labeled anti-hcg-β monoclonal antibody

A pyrene-labeled anti-hcg-β monoclonal antibody was prepared using an anti-hcg-β monoclonal antibody and succinimide diester pyrenebutylate (SPB) (both of them are obtained from Molecular Probes Inc. in the following manner.

Phosphate buffered saline (PBS) (ph 7.4) solution (1000 µl) containing an anti-hcg-β monoclonal antibody at a concentration of 2.0 mg/ml and a solution (20 µl) obtained by dissolving succinimide diester pyrenebutylate (of a 10 times larger amount than that of the antibody) in DMSO so as to contain succinimide diester pyrenebutylate at a concentration of 1.00 mg/ml were mixed. By incubating the mixture at a temperature of 25C° for four hours with stirring it, the anti-hcG-β monoclonal antibody and succinimide diester pyrenebutylate were brought into reaction. An obtained solution was provided into a Sephadex G-25 gel filtration column (Pharmacia) (size: 10 × 60mm, flow rate: about 2 ml/min) so that unreacted SPB was removed and a fraction containing the pyrene-labeled anti-hcG-β monoclonal antibody was recovered.

First, the amount of the prepared pyrene-labeled anti-hcG-β monoclonal antibody which was labeled and characteristics of fluorescent light were evaluated for a recovered fraction.

Measurements performed using a ultraviolet-visible spectrometer (UV-1600PC by Shimazu) showed that the recovered fraction contained the antibody of about 0.8 mg per 1 ml, and pyrene had an optical absorbance of 330 nm indicating that the concentration thereof was 0.88 mg/ml. Therefore, it was confirmed that about 1.1 pyrene was labeled per one molecule of the anti-hcG-β monoclonal antibody. Moreover, as a result of a measurement of characteristics of fluorescent light emitted from the recovered fraction with a fluorometer (RF-5300 PC by Shimazu), it was confirmed that pyrene bound to the anti-hcG-β monoclonal antibody was excited by light having a wavelength of 330 nm to emit fluorescent light having a wavelength of 397 nm and the lifetime of the fluorescent light was 100 ns.

### 2. Preparation of anti-hcG-α monoclonal antibody immobilized cell

As a measurement cell, a polystyrene cell was used. An anti-hcG-α monoclonal antibody 1 mg/ml solution of 700 µl was added to the cell, and then the mixture of the cell and solution was left to stand over a night. Thereafter, after having drawn the solution in the cell to remove it from the cell, the cell was washed. Furthermore, a 1% BSA solution was added to the cell and then the cell were left to stand over a night, so that each inner wall of the cell was blocked by BSA. When a maker enzyme hcg was added to the cell, color development occurred. Thus, it was confirmed that the antibody was immobilized on the inner walls of the cell.

### 3. Measurement of fluorescence polarization degree

As a fluorescence polarization measurement apparatus, FP715 by JASCO Corporation (measuring conditions: measurement temperature of 35 C°, excitation wavelength of 330 nm, fluorescent light wavelength of 397 nm, Gfactor: 0.942).

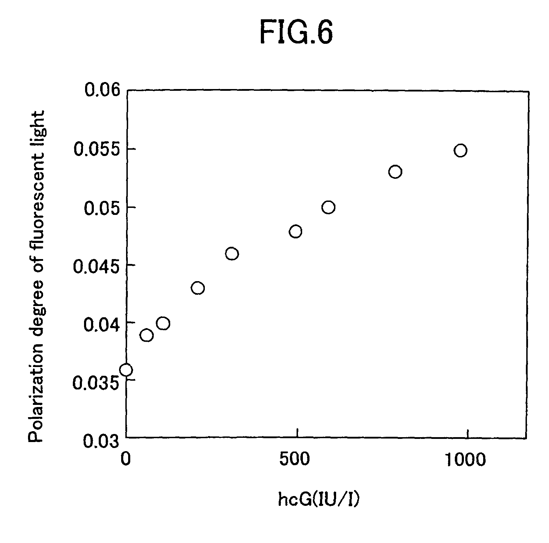

The anti-hcG-α monoclonal antibody immobilized cell prepared in the manner shown in 2 was set in the fluorescence polarization measurement apparatus, and then as samples, solutions containing hcg of 0 IU/l, 50 IU/l, 100 IU/l, 200 IU/l, 300 IU/l, 500 IU/l, 600 IU/l, 800 IU/l and 1000 IU/l, respectively, were prepared. After having mixed the mixture of the hcg solution and the pyrene-labeled anti-hcG-β monoclonal antibody (700 µl) and the each of the hcg solutions (60 µl) and then stirred the obtained mixture at a temperature of 35 C° for 0.5 minute, the fluorescence polarization degree was measured for 0.5 minutes for each of the hcg solutions under the above-described measurement conditions. Measurement results are shown in FIG. 6. As shown in FIG. 6, the results indicate that the fluorescence polarization degree varies almost proportionally to the concentration of hcg.

### INDUSTRIAL APPLICABILITY

The present invention is useful in the fields of environmental measurement, food management, and medical diagnosis.

## Claims

1. A fluorescence polarization assay for measuring a subject substance, comprising the steps of:
(a) preparing a first solid support body on which a binding substance capable of specifically binding to a subject substance is immobilized, and a fluorescent labeled substance which is capable of specifically binding to the subject substance and is labeled with a fluorescent dye;
(b) bringing the fluorescent labeled substance and the binding substance into contact with the subject substance; and
(c) measuring the polarization degree of fluorescent light from the fluorescent labeled substance.

2. The fluorescence polarization assay of claim 1, wherein the subject substance is contained in a solution.

3. The fluorescence polarization assay of claim 1, wherein in the step (c), the polarization degrees of fluorescent light emitted from the fluorescent labeled substance before and after the step (b) are measured.

4. The fluorescence polarization assay of claim 1, further comprising, before the step (b), a step (f) of bringing a solution which does not contain the subject substance into contact with the fluorescent labeled substance and the binding substance
wherein in the step (c), the polarization degrees of fluorescent light from the fluorescent labeled substance after the steps (b) and (f) are measured.

5. The fluorescence polarization assay of claim 4, further comprising a step (g) of obtaining the relationship between each of the polarization degrees of fluorescent light from the fluorescent labeled substance after the steps (b) and (f) and the concentration of the subject substance.

6. The fluorescence polarization assay of claim 1, further comprising:
a step (d) of preparing a second solid support body on which the binding substance is not immobilized, and a fluorescent labeled substance which is capable of specifically binding to the subject substance and is labeled with a fluorescent dye; and
a step (e) of bringing the second solid support body and the fluorescent labeled substance into contact with the subject substance
wherein the second solid support body is made of the same material as a material of the first solid support body from which the binding substance is removed, and
wherein in the step (c), the polarization degrees of fluorescent light from the fluorescent labeled substance after the steps of (b) and (e) are measured.

7. The fluorescence polarization assay of claim 1, wherein the first solid support body is made of a microtiter plate.

8. The fluorescence polarization assay of claim 1, wherein the first solid support body is made of a microparticle.

9. The fluorescence polarization assay of claim 1, wherein the fluorescent labeled substance is labeled with a fluorescent dye which emits fluorescent light whose relaxation time is 1 ns or more.

10. The fluorescence polarization assay of claim 9, wherein the fluorescent dye is bound to a primary amino group, a secondary amino group, a tertiary amino group, a carboxyl group, a thiol group, a phenol group or a hydroxyl group contained in the binding substance.

11. The fluorescence polarization assay of claim 1, wherein the binding substance is any one of an antibody, a receptor, a nucleic acid and an inhibitor.

12. The fluorescence polarization assay of claim 1, wherein the binding substance is any one of a polyclonal antibody, a monoclonal antibody, a chimera antibody, an Fab antibody, an F(ab2) antibody, or an Fv antibody.

13. The fluorescence polarization assay of claim 1, wherein the subject substance is an in vivo substance, a microorganism, or a virus.

14. The fluorescence polarization assay of claim 1, wherein the binding substance is a first antibody,
wherein the fluorescent labeled substance is a second antibody labeled with a fluorescent dye, and
wherein the first and second antibodies recognize different epitopes, respectively.

15. A kit used in a fluorescence polarization assay for measuring a subject substance, comprising:
a solid support body on which a binding substance capable of specifically binding to the subject substance is immobilized; and
a fluorescent labeled substance which is capable of specifically binding to the subject substance and is labeled with a fluorescent dye.

16. A biosensor comprising a flow pass through which a solution containing a subject substance flows
wherein the flow pass includes:
a sample introduction unit for introducing a solution containing a subject substance;
a fluorescent labeled substance holding unit which is connected to the sample introduction unit and in which a fluorescent labeled substance which is capable of specifically binding to the subject substance and is labeled with a fluorescent dye is filled in a state where the fluorescent labeled substance can elute; and
a binding substance holding unit which is connected to the fluorescent labeled substance holding unit and in which a binding substance capable of specifically binding to the subject substance is immobilized on a solid support body.

17. The biosensor of claim 16, wherein the solution is driven by capillary force, centrifugal force, or a potential difference through the flow pass.

18. The biosensor of claim 16, wherein the flow pass is made of the solid support body, and
wherein the solid support body is a capillary.

19. The biosensor of claim 18, wherein the capillary is filled with a carrier.

20. The biosensor of claim 18, wherein in the capillary, a solution flows freely.

21. The biosensor of claim 16, wherein the solid support body is a material which impregnates a solvent of the solution containing the subject substance.

22. The biosensor of claim 16, wherein the biosensor includes at least two flow passes.
